Europäisches Patentamt
European Patent Office
Office européen des brevets

(19)

(11) Publication number: **0 162 422 A2**

(12)

# EUROPEAN PATENT APPLICATION

(21) Application number: 85106066.5

(22) Date of filing: 17.05.85

(51) Int. Cl.[4]: C 12 P 21/02
A 61 K 37/02, C 12 N 1/20
//(C12P21/02, C12R1:465),
(C12N1/20, C12R1:465)

(30) Priority: 21.05.84 JP 100441/84

(43) Date of publication of application: 27.11.85 Bulletin 85/48

(84) Designated Contracting States: AT BE CH DE FR GB IT LI LU NL SE

(71) Applicant: MICROBIAL CHEMISTRY RESEARCH FOUNDATION
14-23, Kamiosaki 3 Chome Shinagawa-ku
Tokyo 141(JP)

(72) Inventor: Umezawa, Hamao, Prof.
23, Toyotama-kita 4-chome
Nerima-ku Tokyo(JP)

(72) Inventor: Takeuchi, Tomio
1-11, Higashigotanda 5-chome
Shinagawa-ku Tokyo(JP)

(72) Inventor: Aoyagi, Takaaki
3-6, Honkugenuma 3-chome
Fujisawa-city Kanagawa Prefecture(JP)

(72) Inventor: Hamada, Masa
26, Naito-ch 1-chome
Shinjuku-ku Tokyo(JP)

(72) Inventor: Ogawa, Keiji
20-9, Kinugaoka 2-chome
Hachioji-city Tokyo(JP)

(72) Inventor: Iinuma, Hironobu
35-6-301, Shirako 3-chome
Wako-city Saitama Prefecture(JP)

(74) Representative: Becker, Heinrich Karl Engelbert, Dr. et al,
HOECHST AKTIENGESELLSCHAFT Central Patent
Department P.O. Box 80 03 20
D-6230 Frankfurt am Main 80(DE)

(54) **The novel physiologically active substance foroxymithine, a process and microorganisms for its production and its use as medicament.**

(57) The present invention relates to the novel physiologically active substance Foroxymithine of the formula

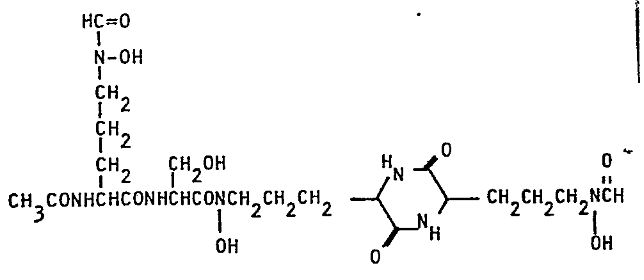

and a microbial process for preparing the same.

The compound shows angiotensin I converting enzyme inhibiting activity as well as antihypertensive activity and can, therefore, be used as medicament.

EP 0 162 422 A2

The present invention relates to a novel physiologically active substance, called foroxymithine and pharmaceutically salts or esters thereof, a process for producing this substance from microorganisms of the genus Streptomyces, to these microorganism strains and to medicaments containing this substance as active ingredient.

Foroxymithine has the following formula

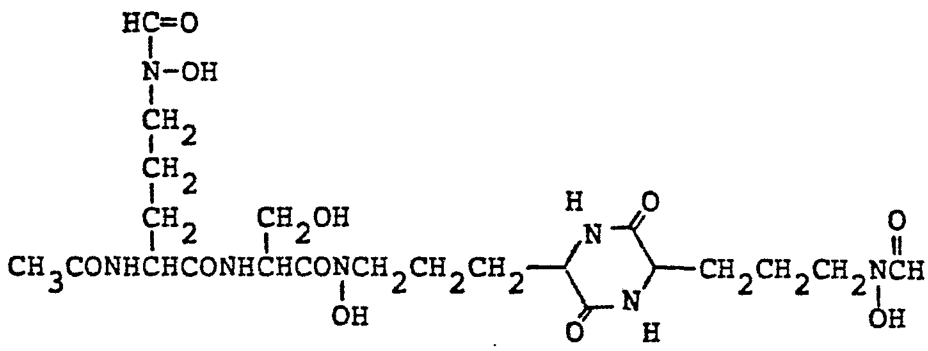

It shows the activity of inhibiting angiotensin I converting enzyme and antihypertensive activity.

Foroxymithine can be produced by cultivating microorganisms of the genus Streptomyces and recovering the substance from the culture medium. Examples of strains which produce foroxymithine are Streptomyces nitrosporeus MG329-CF 56 isolated from a soil example collected in the yard of the Institute of Microbial Chemistry in Tokyo, and Streptomyces zaomyceticus MG325-CF 7 isolated from the soil of Mount Kurama located at Sakyo-ku, Kyoto.

These strains were deposited on Sept. 26, 1983 with the Fermentation Research Institute, Agency of Industrial Science and Technology, MITI, where they were assigned FERM-P Nos. 7244 and 7243, respectively.

The physical properties of foroxymithine are as follows.

Foroxymithine dihydrate has a melting point of 105 - 130°C (decomposition temperature), and a molecular weight, determined by mass spectrometry, of 575. The elemental analysis values are C: 43.46%, H: 6.31%, N: 16.12%,

defining the molecular formula as $C_{22}H_{37}N_7O_{11}\cdot 2H_2O$. The specific rotation is $[\alpha]^{22}$ -44.5° (c=1,$H_2O$). The infrared absorption spectrum of foroxymithine by the KBr method is as shown in the attached drawing, exhibiting characteristic absorptions at 3400, 2350, 2940, 1660, 1540, 1460, 1380, 1340, 1240, 1060, and 880 $cm^{-1}$ (see attached figure).

The proton NMR spectrum (in heavy water, 100 MHz) of foroxymithine shows absorptions at 2.0-2.4 ($CH_2$ x 6), 2.5 ($CH_3$ x 1), 3.9-4.25 ($CH_2$ x 3), 4.25-4.4 ($CH_2$ x 1), 4.5-4.75 (CH x 2), 4.75-4.9 (CH x 1), 5.46-5,65 (CH x 1), 8.42 (=C-H x 1.5), 8.78 (=C-H x 0.5) ppm.

The novel microorganism strains MG329-CF 56 (FERM-P No. 7244) and MG325-CF7 (FERM-P No. 7243) have the following microbiological characteristics:

(1) Microbiological characteristics of MG329-CF56 (FERM-P No. 7244)

(A) Morphology

Microscopically, substrate mycelia were branched and extended relatively long, straight aerial hyphae (rectiflexibles). On the aerial hyphae, neither spirals nor whirls were observed. Chains of mature spores included those having more than 10 spores. The spores ranged in size from about 0.4 to 0.6 x 0.8 to 1.4 microns, and their surfaces were smooth.

(B) Culture characteristics on various media

In the following disclosure, the standards indicated in square brackets [ ] to describe colors comply with the Color Harmony Manual adopted by the container corporation of America.

(a) On sucrose nitrate agar medium (cultured at 27°C): Growth was colorless, and brownish gray aerial mycelium developed slightly on the growth; no soluble pigment.

(b) On glucose-asparagine agar medium (cultured at 27°C):

Growth was colorless, and no aerial mycelium developed; no soluble pigment.

(c) On glycerol-asparagine agar medium (ISP-5, cultured at 27°C):

Growth was pale yellow [1 1/2 ie, Lt. Olive], and white to light brownish gray [2 fe, covert Gray] aerial mycelium developed on the growth; slightly yellowish soluble pigment was produced.

(d) On starch-inorganic salts agar medium (ISP-4, cultured at 27°C):

Growth was pale yellow [1 1/2 ea, Lt. Yellow], and white to light brownish gray [2 dc, Natural String] aerial mycelium developed on the growth; slightly yellowish soluble pigment was produced.

(e) On tyrosine agar medium (ISP-7, cultured at 27°C):

Growth was pale yellowish brown, and white to light gray [2 fe, covert Gray] aerial mycelium developed on the growth; slightly brown soluble pigment was produced.

(f) On nutrient agar medium (cultured at 27°C):

Growth was olive gray [1 ig, Olive Gray], and brownish white [3 ba, Pearl Shell Tint to 3 dc, Natural] aerial mycelium developed on the growth; brown soluble pigment was produced.

(g) On yeast extract-malt extract agar medium (ISP-2, cultured at 27°C):

Growth was pale yellowish brown, and white to light brownish gray [2 fe, covert Gray to 3 fe, Silver Gray] aerial mycelium developed on the growth; no soluble pigment was produced.

(h) On oatmeal agar medium (ISP-3, cultured at 27°C):

Growth was pale yellowish brown, and white to light brownish gray [3 fe, Silver Gray] aerial mycelium developed on the growth; no soluble pigment was produced.

(i) Glycerol-nitrate agar medium (cultured at 27°C):

Growth was pale yellow [1 1/2ea, Lt. Yellow], and white to light brownish gray [2 dc, Natural String] aerial mycelium developed on the growth; yellowish soluble pigment was produced.

(j) On starch agar medium (cultured at 27°C):

Growth was pale yellow, and brownish white to light brownish gray [2 fe, covert Gray to 3 fe, Silver Gray] aerial mycelium developed on the growth; no soluble pigment was produced.

(k) On calcium malate agar medium (cultured at 27°C):

Growth was pale yellow [1 ca, Pale Yellow], and white to light aerial mycelium developed on the growth; no soluble pigment was produced.

(l) On cellulose medium (cultured at 27°C):

No growth was observed after 21 days of culture.

(m) On gelatin stab culture medium:

On simple gelatin medium (cultured at 20°C), growth was pale yellow; light gray to light brownish gray aerial mycelium developed on the growth; and pale brown soluble pigment was produced. On glucose peptone gelatin medium (cultured at 27°C), growth was pale brown; white aerial mycelium developed on the growth; and brown soluble pigment was produced.

(n) On skimmed milk medium (cultured at 37°C):

Growth was colorless, and no aerial mycelium developed and no soluble pigment was produced.

(C) Physiological properties

(a) growth temperature:

Tests for growth were carried out at temperatures of 20, 24, 27, 30, 37 and 50°C on yeast-starch agar medium (pH 7.0) comprising 1% soluble starch, 0.2% yeast extract (a product of Daigo Eiyo Kagaku) and 2.0% agar. Growth occured at all these temperatures, except 50°C. The optimum temperature was considered to be about 27 to 30°C.

(b) Liquefaction of gelatin (15% simple gelatin: cultured at 20°C, glucose peptone gelatin: cultured at 27°C):

On each medium liquefaction began after about 5 days of culture. The liquefactive strength was intermediate to high.

(c) Hydrolysis of starch (tested on starch-inorganic salts agar medium, and starch agar medium, each cultured at 27°C):

Hydrolysis was observed after about 3 days of cultivation. The hydrolytic strength was high.

(d) Coagulation and peptonization of skimmed milk (cultured at 37°C):

Coagulation began after about 4 days of cultivation. Immediately, coagulation began and was completed virtually at 14 to 21 days. The strength was intermediate to high.

(e) Production of melanoid pigment (tested on tryptone-yeast extract broth (ISP-1), peptone-yeast extract-iron agar medium (ISP-6), tyrosine agar medium (ISP-7), each cultured at 27°C):

No melanoid pigment was produced on any of the media.

(f) Utilization of carbon sources (tested on Pridham-Gottlieb agar medium (ISP-9) cultured at 27°C):

Glucose and L-arabinose were utilized. D-xylose and L-rhamnose were evaluated to be probably utilized. D-fructose was evaluated probably not to be utilized. Sucrose, inositol, raffinose or D-mannitol was not utilized.

(g) Dissolution of calcium malate (tested on calcium malate agar medium, cultured at 27°C):

Calcium malate was dissolved around the growth after about 5 days of cultivation. The dissolving strength was intermediate.

(h) Reduction of nitrate (tested on 0.1% potassium nitrate-containing broth (ISP-8), cultured at 27°C):

Reduction was positive.

The characteristics of the strain MG329-CF56 described above can be summarized as follows:

Morphologically, no whirls are seen and no spirals are formed on aerial hyphae. The surfaces of spores are smooth. Growths on various media are colorless to pale yellow or pale yellowish brown, and white to light brownish gray aerial mycelia develop on the growths. Yellow to brownish soluble pigments are produced. Melanoid pigment production is negative, and proteolytic activity is moderate to marked. The activity of hydrolyzing starch is also marked. Furthermore, the 2,6-diaminopimelic acid contained in the cell wall of this strain is of the LL-type, showing the strain to belong to the genus Streptomyces.

Search on known species based on these properties has shown the strain MG329-CF56 to be closely related to S. nitrosporeus (The International Journal of Systematic Bacteriology, Vol. 18, page 152, 1968 - Reference 1; The Journal of Antibiotics, Series A, Vol. 5, page 477, 1952 - Reference 2; Waksman, The Actinomycetes, Vol. 2, page 248, 1961 - Reference 3) and S. griseolus (The International Journal of Systematic Bacteriology, Vol. 18, page 122,

1968 - Reference 1; Waksman, The Actinomycetes, Vol. 2, page 222, 1961 - Reference 2). S. griseolus liquefies gelatin mildly, coagulates milk slowly, and hydrolyzes starch weakly. Furthermore, this species utilizes D-fructose as a carbon source. These properties clearly distinguish S. griseolus from MG329-CF56.

Therefore, the properties of MG329-CF56 and S. nitrosporeus will be compared below.

| | MG329-CF56 | Streptomyces nitrosporeus |
|---|---|---|
| Shape of aerial hypha | Rectiflexibilities | Rectiflexibilities |
| Surface of spore | Smooth | Smooth |
| Color or aerial mycelium | Gray color-series | Gray color-series |
| Color of growth | Colorless to pale yellow or pale yellowish brown | Colorless (Grayish yellow to yellowish brown) |
| Soluble pigment | No production to yellow or brown | No production or yellowish or brownish gray |
| Production of melanoid pigment | – | – |
| Decomposition of cellulose | – | +* |
| Hydrolysis of starch | + strong | +* strong |
| Coagulation of milk | + strong | +* strong |
| Peptonization of milk | + medium to strong | +* strong |
| Liquefaction of gelatin | + medium to strong | +* strong |
| Reduction of nitrate | + | +* |
| Utilization of carbon sources | | |
| Glucose | + | + |
| L-arabinose | + | + |
| D-xylose | | + |
| D-fructose | | Variable |
| Sucrose | – | – |
| Inositol | – | – |
| L-rhamnose | | + |
| Raffinose | – | – |
| D-mannitol | – | – |

Notes: + stands for probably negative utilization, and probably positive utilization.
Of the properties of Streptomyces nitrosporeus, those marked * refer to the results disclosed in Reference 2 mentioned earlier in regard to this species, and the other properties concern the results disclosed in Reference 2.

As shown in the above table, the greatest difference between the strain MG329-CF56 and Streptomyces nitrosporeus is in the decomposition of cellulose. Although some minor differences exist between the two microorganisms, both are very closely related to each other, and the shapes of their spores quite resemble photomicrographically.

Accordingly, MG329-CF56 was identified as Streptomyces nitrosporeus MG329-CF56.

(2) Microbiological characteristics of MG325-CF7 (FERM-P No. 7243)

(A) Morphology

Microscopically, substrate mycelia of MG325-CF7 were branched and extended relatively long, straight aerial hyphae or hooked aerial hyphae (hooks). On the aerial hyphae, no spirals were formed, and no whirls were observed. Chains of mature spores included those having more than 10 spores. The spores ranged in size from about 0.4 to 0.8 x 0.8 to 1.0 micron, and their surfaces were smooth.

(B) Culture characteristics on various media

In the following disclosure, the standards indicated in square brackets [ ] to describe colors comply with the Color Harmony Manual adopted by the Container Corporation of America.

(a) On sucrose nitrate agar medium (cultured at 27°C):

Growth was pale yellow [2 ca, Lt. Ivory to 2 le, Mustard], and white aerial mycelium developed slightly on the growth; no soluble pigment.

(b) On glucose-asparagine agar medium (cultured at 27°C):

Growth was pale yellow [1 1/2 ea, Lt. Yellow], and white to grayish white [c, Lt. Gray] aerial mycelium developed slightly on the growth; no soluble pigment.

(c) On glycerol-asparagine agar medium (ISP-5, cultured at 27°C):

Growth was pale yellow to pale yellowish brown, and white to grayish white aerial mycelium developed on the growth; no soluble pigment was produced.

(d) On starch-inorganic salts agar medium (ISP-4, cultured at 27°C):

Growth was pale yellow [2 gc, Bamboo], and white to yellowish gray [2 ea, Lt. Wheat] or light gray [3 fe, Silver Gray] aerial mycelium developed abundantly on the growth; yellowish soluble pigment was produced.

(e) On tyrosine agar medium (ISP-7, cultured at 27°C):

Growth was pale brown [3 ng, Yellow Maple] to grayish yellowish brown [3 pl, Deep Brown], and grayish white to brownish white [3 ca, Pearl Pink] aerial mycelium developed abundantly on the growth; brownish soluble pigment was produced.

(f) On nutrient agar medium (cultured at 27°C):

Growth was light brownish gray [3 le, Cinnamon], and brownish grayish white to light gray aerial mycelium developed on the growth; brown soluble pigment was produced.

(g) On yeast extract-malt extract agar medium (ISP-2, cultured at 27°C):

Growth was pale yellow [2 ne, Mustard Gold], and white to grayish white or yellowish gray aerial mycelium developed on the growth; no soluble pigment was produced.

(h) On oatmeal agar medium (ISP-3, cultured at 27°C):

Growth was pale yellow [1 fb, Pastel Yellow], and white to light gray aerial mycelium developed on the growth; yellow soluble pigment was produced.

(i) Glycerol-nitrate agar medium (cultured at 27°C):

Growth was pale yellow [2 ea, Lt. Wheat], and white aerial mycelium developed slightly on the growth; no soluble pigment was produced.

(j) On starch agar medium (cultured at 27°C):

Growth was colorless to pale yellow [1 1/2 ea, Lt. Yellow], and no aerial mycelium developed; no soluble pigment was produced.

(k) On calcium malate agar medium (cultured at 27°C):

Growth was colorless to pale yellow, and white aerial mycelium developed slightly on the growth; no soluble pigment was produced.

(l) On cellulose medium (filter paper-containing synthetic liquid, cultured at 27°C):

No growth was observed after 21 days of cultivation.

(m) On gelatin stab culture medium:

On simple gelatin medium (cultured at 20°C), growth was colorless to pale yellow, white aerial mycelium developed slightly on the growth; and brown soluble pigment was produced. On glucose peptone gelatin medium (cultured at 27°C), growth was colorless to pale yellow; no aerial mycelium developed, dark brown soluble pigment was produced.

(n) On skimmed milk medium (cultured at 37°C):

Growth was colorless, and no aerial mycelium developed and brownish soluble pigment was produced.

(C) Physiological properties

(a) Growth temperatures:

Tests for growth were carried out at temperatures of 20, 24, 27, 30, 37 and 50°C on

starch-yeast agar medium (pH 7.0) containing 1.0% of soluble starch, 0.2% of yeast extract (a product of Daigo Eiyo Kagaku), and 3.0% of agar. Growth was good at 20, 24, 27 and 30°C, slight at 37°C, and negative at 50°C. The optimum temperature was considered to be about 27 to 30°C.

(b) Liquefaction of gelatin (15% simple gelatin; cultured at 20°C; glucose-peptone gelatin; cultured at 27°C):

On simple gelatin medium and glucose-peptone gelatin medium liquefaction began after about 7 days of cultivation. The liquefactive strength was medium to low.

(c) Hydrolysis of starch (tested on starch-inorganic salts agar medium, and starch agar medium, each cultured at 27°C):

Hydrolysis was observed after about 3 days of cultivation on starch-inorganic salts agar medium, and after about 5 days of cultivation on starch agar medium. The hydrolytic strength was medium to high.

(d) Coagulation and peptonization of skimmed milk (skimmed milk, cultured at 37°C):

Coagulation began after about 7 days of cultivation, and was immediately completed. At this time, peptonization began. The strength was medium.

(e) Production of melanoid pigment (tested on tryptone-yeast extract broth (ISP-1), peptone-yeast extract-iron agar medium (ISP-6), tyrosine agar medium (ISP-7), each cultured at 27°C):

Melanoid pigment was produced on any of these media, but its production was weak on tyrosine agar medium.

(f) Utilization of carbon sources (tested on Pridham-Gottlieb agar medium (ISP-9), cultured at 27°C):

Glucose and D-xylose were utilized, but sucrose, inositol, L-rhamnose, raffinose and D-mannitol were not utilized. L-arabinose or D-fructose was probably not utilized.

(g) Dissolution of calcium malate (tested on calcium malate agar medium cultured at 27°C):

Calcium malate was dissolved around the growth after about 10 days of cultivation. The dissolving strength was medium.

(h) Reduction of nitrate (tested on 0.1% potassium nitrate-containing peptone water (ISP-8), cultured at 27°C:

Reduction was positive.

The characteristics of MG325-CF7 described above can be summarized as follows:

Morphologically, no sporangium is observed, no spirals are formed on aerial hyphae, and no whirls are seen. The surfaces of spores are smooth. Growths on various media are colorless to pale yellow or yellowish brown. White to light gray or yellowish gray aerial mycelia develop on the growths. Soluble pigment are yellow or brownish. Melanoid pigment production is positive.

Proteolytic activity is medium. Hydrolysis of starch is medium to strong. The 2,6-diaminopimelic acid contained in the cell wall of this strain is of the LL-type, showing the strain to belong to Streptomyces.

Search on known species based on the above-mentioned properties has shown MG 325-CF7 to be related most closely to Streptomyces zaomyceticus (The Internation Journal of Systematic Bacteriology, Vol. 22, page 374, 1972 - Reference 1; The Journal of Antibiotics Series A, Vol. 7, page 134, 1954 - Reference 2).

The properties of MG325-CF7 and Streptomyces zaomyceticus IMC S-0663 (ISP5196) have been compared, and the results will be summarized below.

| | MG325-CF7 | Streptomyces zaomyceticus IMC S-0663 (ISP5196) |
|---|---|---|
| Shape of aerial hypha | Straight or hooked | Straight or hooked |
| Surface of spore | Smooth | Smooth |
| Color of aerial mycelium | White to light gray or yellowish gray | White to light gray or yellowish gray |
| Color of growth | Colorless to pale yellow or pale yellowish brown | Colorless to pale yellow or pale yellowish brown |
| Soluble pigment | Colorless to yellowish brownish | Colorless to brownish |
| Production of melanoid pigment | | |
| ISP-1 | + | + |
| ISP-6 | + | + |
| ISP-7 | ∓ very weak | − |
| Hydrolysis of starch | + medium to strong | + medium to strong |
| Coagulation of milk | + | + strong* |
| Peptonization of milk | + medium | + medium to strong* |
| Liquefaction of gelatin | | |
| Simple gelatin | + medium to weak | + medium to weak |
| Glucose-peptone-gelatin | + medium to weak | + medium to weak |
| Reduction of nitrate | + | + |
| Utilization of carbon sources | | |
| Glucose | + | + |
| L-arabinose | − | ± +o |
| D-xylose | + | + |
| D-fructose | − | − |
| Sucrose | − | − |
| Inositol | − | − |
| L-rhamnose | − | − |
| Raffinose | − | − |
| D-mannitol | − | − |

Notes: +: Positive
−: Negative
The symbol ± in the Utilization of carbon sources means that the carbon source was probably utilized. Of the properties of Streptomyces zaomyceticus, those marked * refer to the data disclosed in the aforementioned Reference 2, and those marked o concern the data disclosed in Reference 1.

As shown in the above table, MG325-CF7 and Streptomyces zaomyceticus have practically the same properties, except for the utilization of L-arabinose, showing that both are very similar species. Accordingly, MG325-CF7 was identified as Streptomyces zaomyceticus MG325-CF7.

The process for producing foroxymithine according to the present invention will be described in detail below.

The cultivation of foroxymithine-producing microorganisms in the process of this invention is carried out using nutritional sources. They may be any known nutritional sources which can be utilized by ordinary microorganisms. For example, commercially available fats and oils and carbohydrates, such as glycerol, glucose, lactose, starch and molasses are useful as carbon sources. Examples of nitrogen sources are peptone, meat extract, corn steep liquor, cotton seed meal, peanut meal, soybean meal, corn gluten meal, fish meal, yeast extract, N-Z amine, casein, sodium nitrate, ammonium nitrate, and ammonium sulfate. Sodium chloride, phosphates, calcium carbonate, and magnesium sulfate can be used as inorganic nutrients. Particularly preferred culture media contain carbon sources such as glycerol and nitrogen sources such as cotton and seed meal. It is preferred to use culture media containing 1.5% glycerol, 1.5% cotton seed meal, 0.3% sodium chloride, and 0.2% L-asparagine monohydrate.

To mass-produce foroxymithine, liquid culture is preferred. The cultivation temperature may be any temperature at which the foroxymithine-producing microorganism is grown to produce foroxymithine. The preferred temperature is 25 to 35°C. The cultivation is continued usually until this substance is produced and accumulated in sufficient amounts in the culture broth.

If the foroxymithine-producing Streptomyces nitrosporeus MG329-CF56 (FERM-P-No. 7244) was used as the starting organism, the production process was carried out as follows: A 500 ml Sakaguchi flask was charged with 120 ml of culture medium (adjusted to pH 7.4) containing 1.5%

glycerol, 1.5% cotton seed meal, 0.3% sodium chloride and 0.2% L-asparagine monohydrate. The culture medium was sterilized, and inoculated with a loopful of a slant culture of the foroxymithine-producing strain Streptomyces nitrosporeus MG329-CF56 (FERM-P No. 7244). The inoculum was shake-cultured aerobically at 27°C. The accumulation of foroxymithine was recognized after 48 to 192 hours of culture.

Foroxymithine can be produced satisfactorily by jar fermentation as in the case of shake culture. For example, 15 liters of a culture medium was placed in a 30-liter fermenter, and sterilized. 0.5 liter of a precultured seed culture broth was inoculated to the culture medium, and the inoculated medium was stirred at 27°C at 200 rpm under aeration with 15 liters of sterile air per minute. Under these conditions, the production of foroxymithine peaked in 68 hours.

Follow-up of foroxymithine during the cultivation step and the purification step was performed in accordance with the determination of anti-angiotensin I converting enzyme activity by the method to be described below.

Anti-angiotensin I converting enzyme activity was determined by a modification of the angiotensin I converting enzyme determination technique described in M. Hayakari et al., Analytical Biochemistry Vol. 84, pages 361-369 (1978). Hippuryl-L-histidyl-L-leucine as a substrate was dissolved at a concentration of 5 mg/ml in Tris-hydrochloride buffer (0.5M, pH 8.0) containing 0.3M sodium chloride. 0.40 ml of a solution containing a test material was added to 0.05 ml of the resulting solution. The mixture was heated at 37°C for 3 minutes. Then, 0.05 ml of a solution containing angiotensin I converting enzyme that had been obtained from a bovine lung and partially purified was added, and the mixture was reacted for 30 minutes at 37°C. 0.03 ml of 1N sodium hydroxide was added to the system to terminate the reaction, and 2 ml of 0.06M sodium phosphate buffer (pH 7.2) was added

after 15 minutes. Then, 2 ml of a methyl cellosolve solution of 1% cyanuric chloride was added for color reaction. After the system was allowed to stand for 15 minutes at room temperature, its absorbance (termed absorbance (a)) at 382 nm was measure. Separately, a mixed solution containing no test material was prepared similarly, and reaction similarly. The reaction system as the control was measured for absorbance, which was termed absorbance (b). The angiotensin I converting enzyme inhibition rate (%) was calculated from the equation (b-a)/b x 100. Pure foroxymithine dihydrate at a concentration of 7 µg/ml inhibited angiotensin I converting enzyme by 50% ($IC_{50}$ = 7 µg/ml).

Next, foroxymithine present in the filtrate of the cultured broth of the foroxymithine-producing organism and in the bacterial cells is recovered from them. Adsorption of this substance from the culture filtrate onto an adsorbent, followed by elution, can give this substance in a good yield. Activated carbon, ion-exchange resins, etc. can be used as adsorbents. For instance, foroxymithine is adsorbed to activated carbon, and eluted with a 50% aqueous solution of acetone. In detail, to the culture filtrate is added 2% by weight, based thereon, of activated carbon, and the mixture is stirred. After washing with water, the system is eluted with a 50% aqueous solution of acetone in an amount of a quarter of the volume of the culture filtrate, thereby to obtain an active fraction. Concentration under reduced pressure gives a crude concentrate of foroxymithine. The crude concentrate is diluted with water, and adsorbed to a column of DEAE-Sephadex® A 25 ($OH^-$)-type packed to 1/60 of the volume of the culture filtrate. After washing with water, the column is eluted with a 0.1M aqueous solution of sodium chloride. After neutralization with hydrochloric acid, the eluate is concentrated under reduced pressure to obtain a crude powder of foroxymithine.

Reversed-phase high-performance liquid chromatography is useful for its purification. Chromatography on Nucleosil ®5C18 (a product of Macherey-Nagel) is particularly effective. The chromatographic column is eluted with methanol-0.4% acetic acid (10:90) to yield pure form of foroxymithine.

The biological activities of foroxymithine in accordance with this invention were studied in terms of its pharmacological actions. The studies showed this substance to have an antihypertensive effect. This effect will be described with reference to the following Experimental Example.

Experimental Example

This Example illustrates the effect of foroxymithine on blood pressure in spontaneous hypertensive rats (SH rats).

SH rats weighing 320 to 358 g were orally administered foroxymithine dihydrate as an aqueous solution at doses of 12.5, 25 and 50 mg/kg. After the administration, blood pressure was measured at certain time intervals. Changes in blood pressure in the foroxymithine-administered group were compared with those in the control group orally administered distilled water only. The results are shown in Table 1.

Table 1 Effect of Foroxymithine on Blood Pressure in Spontaneous Hypertensive Rats

| Test compound | Oral dose (mg/kg) | | Mean blood pressure ± S.D. (mmHg) and decrease in blood pressure (%) following administration | | | | |
|---|---|---|---|---|---|---|---|
| | | | Before Administration | After administration | | | |
| | | | | 1 hour | 3 hours | 6 hours | 24 hours |
| Control (distilled water) | - | Mean b.p.* | 213 ± 24.7 | 208 ± 17.6 | 208 ± 17.6 | 203 ± 17.6 | 215 ± 7.0 |
| | | Decrease in b.p. | - | 2.3 | 2.3 | 4.7 | -0.9 |
| Foroxymithine dihydrate | 12.5 | Mean b.p. | 210 ± 0.7 | 180 ± 0.7 | 190 ± 28.2 | 200 ± 14.1 | 210 ± 14.1 |
| | | Decrease in b.p. | - | 14.3 | 9.5 | 4.8 | 0 |
| | 50.0 | Mean b.p. | 210 ± 0.0 | 160 ± 7.0 | 153 ± 3.5 | 193 ± 10.6 | 213 ± 3.5 |
| | | Decrease in b.p. | - | 23.8 | 27.1 | 8.1 | -1.4 |

* blood pressure

The above results demonstrate foroxymithine to lower blood pressure in spontaneous hypertensive rats (SH rats).

Foroxymithine obtained by this invention is thus useful as an antihypertensive agent.

Acute toxicity studies in mice showed foroxymithine dihydrate, 800 mg/kg i.v., to cause no deaths. Foroxymithine is therefore a low-toxicity substance.

Accordingly, a third gist of this invention lies in providing an antihypertensive agent containing as its active principle the substance foroxymithine.

Antihypertensive agents in accordance with this invention can be prepared by blending foroxymithine or its pharmaceutically acceptable salts or esters with customarily used carriers. Various chemotherapeutic agents may be further incorporated into the resulting products.

Examples of the salts of foroxymithine are salts formed between the hydroxam group of foroxymithine and pharmaceutically acceptable cations, such as alkali metal ions, alkaline earth metal ions and other suitable metal ions.

The esters of foroxymithine include, for example esters formed between the hydroxyl group of foroxymithine and organic acids such as formic acid and acetic acid or other suitable acids.

The compounds or pharmaceuticals in accordance with this invention may be administered as oral preparations, injections or rectal suppositories. The injections are prepared by adding pH regulators, buffers, stabilizers, excipients, etc. to said compounds as active ingredients. The mixture are freeze-dried by customary methods to prepare lyophilized injections.

Alternatively, pH regulators, buffers, stabilizers, isotonizers, local anesthetics, etc. are added to the compounds as active ingredients, and the mixtures are processed by customary methods to prepare hypodermic, intramuscular, and intravenous injections. Solid preparations for oral administration can be prepared by adding

excipients, and if desired, binders, disintegrators, lubricants, colorants, taste correctors, odor correctors, etc. to said compounds as active ingredients, and then processing the mixtures by customary methods to prepare tablets, coated tablets, granules, powders, capsules, etc. Liquid preparations for oral administration can be prepared by adding taste correctors, buffers, stabilizers, odor correctors, etc. to those compounds as active ingredients, and then processing the mixtures by customary methods to prepare syrups and dry syrups. In the preparation of rectal suppositories, excipients, and if desired, surfactants are added to said compounds as active ingredients, and the mixtures are processed by customary methods to produce the suppositories.

Doses of foroxymithine-containing preparations vary with symptoms. In adults, their usual doses are 0.02 to 2,000 mg, 3 times daily, as foroxymithine.

The production of foroxymithine in accordance with this invention will be described in more detail with reference to the following Examples. This invention is in no way limited to these Examples, however, since the physicochemical properties of this substance, as well as its production and purification processes have been elucidated by the present inventors, thus making it easy to modify the processes disclosed in this Specification.

Example 1

A culture medium was prepared according to the following recipe:

| | |
|---|---|
| Glycerol | 1.5% |
| Cotton seed meal | 1.5% |
| Sodium chloride | 0.3% |
| L-asparagine monohydrate | 0.2% |
| pH before sterilization | 7.4 |

A loopful of spores and mycelium from a slant culture of a foroxymithine-producing strain, Streptomyces nitrosporeus MG329-CF56 (FERM-P-7244), was inoculated to each of 500 ml Sakaguchi flasks each containing 120 ml of the above medium which had been sterilized for 20 minutes at 120°C. The flasks were placed on a reciprocating shaker, and the inoculated medium was aerobically cultured at 27°C at 130 reciprocations per minute. The amount of foroxymithine produced was investigated at certain time intervals. The concentration of foroxymithine determined by the angiotensin I converting enzyme inhibitory activity was maximal after 3 days of cultivation and stable until 12 days after cultivation was begun. 120 ml of the above medium which had been sterilized for 20 minutes at 120°C. The flasks were placed on a reciprocating shaker, and the inoculated medium was aerobically cultured at 27°C at 130 reciprocations per minute. The amount of foroxymithine produced was investigated at certain time intervals. The concentration of foroxymithine determined by the angiotensin I converting enzyme inhibitory activity was maximal after 3 days of cultivation and stable until 12 days after cultivation was begun.

Example 2

A foroxymithine-producing strain, Streptomyces nitrosporeus MG329-CF56 (FERM-P 7244) was cultured for 72 hours under the cultivation conditions shown in Example 1 to prepare a seed culture broth. 5 ml of the seed culture broth was inoculated to the same medium as disclosed in Example 1, and the inoculated medium was cultured similarly for 72 hours. 5 Liters of the resulting broth was filtered with diatomaceous earth used as a filter aid, and washed with water to obtain 5.5 liters of the filtrate. The angiotensin I converting enzyme inhibitory activity ($IC_{50}$) of this filtrate was 10 µg/ml. To 5.5 liters of the culture filtrate was added 100 g of activated carbon for chromatography (a product of Wako Pure Chemical), and the mixture was stirred to adsorb foroxymithine to activated carbon.

The activated carbon was collected by filtration, and washed with 2 liters of water. The washed carbon was stirred for 30 minutes in 2.4 liters of a 50% aqueous solution of acetone to elute foroxymithine. The system was filtered, and the filtrate was concentrated to dryness under reduced pressure to obtain 15.8 g of a crude powder ($IC_{50}$ = 416 µg/ml). The resulting crude powder was dissolved in 500 ml of deionized water, and the solution was adsorbed to a 250 ml column of DEAE Sephadex ®A25 ($OH^-$ type). The column was washed with deionized water, and eluted with 1 liter of a 0.1M aqueous solution of sodium chloride. The eluate was neutralized with hydrochloric acid, and concentrated to dryness under reduced pressure to obtain 11.2 g of a crude powder ($IC_{50}$ = 32 µg/ml). The crude powder was dissolved in 100 ml of deionized water, and the solution was adsorbed to a 600 ml column of reversed-phase silica gel (Kieselgel 60, silanized, a product Merck), followed by developing the column with deionized water. The active fractions were combined, and concentrated to dryness under reduced pressure to obtain 3.29 g of a crude powder ($IC_{50}$ = 12.4 µg/ml). The crude powder was dissolved in a mixture of 200 ml of 99.5% ethanol and 50 ml of 25% aqueous ammonia. The solution was adsorbed to a 250 ml silica gel column which had been equilibrated with 99.5% ethanol-25% aqueous ammonia (4:1), and was then developed with the same solvent mixture. The active fractions were combined and concentrated to dryness under reduced pressure to obtain 1.96 g of a crude powder ($IC_{50}$ = 11 µg/ml). The crude powder was dissolved in 30 ml of a 0.4% aqueous solution of acetic acid, and 30 ml of the solution was adsorbed to a high-performance liquid chromatographic column, Nucleosil® 5018, 20 mm in diameter and 300 mm in length (a product of Marchery-Nagel), with 10 ml of the solution being adsorbed at a time.

The column had been equilibrated with a 10:90 mixture of methanol and an aqueous solution of 0.4% acetic acid. The column was eluted with the same mixture at a

flow rate of 6 ml/minute. Active fractions were collected in amounts of 6 ml each, and combined and concentrated to dryness. The dry concentrate was dissolved in 100 ml of distilled water, and lyophilized to obtain 1.23 g of a white powder. The resulting foroxymithine dihydrate had a angiotensin I converting enzyme inhibitory activity ($IC_{50}$) of 7 µg/ml.

Example 3

A medium was prepared according to the following recipe:

| | |
|---|---|
| Glycerol | 1.5% |
| Cotton seed meal | 1.5% |
| Sodium chloride | 0.3% |
| Monosodium L-glutamate | 0.5% |
| pH before sterilization | 7.4 |

A loopful of spore and mycelium from a slant culture of a foroxymithine-producing strain, Streptomyces zaomyceticus MG 325-CF7 (FERM P-7243) was inoculated to each of 500 ml Sakaguchi flasks each containing 120 ml of the above medium which had been sterilized for 20 minutes at 120°C. The flasks were placed on a reciprocating shaker, and the inoculated medium was aerobically cultured at 27°C at 130 reciprocations per minute. The cultured broth after 3 days of cultivation was inoculated in amounts of 0.5 liter eah into four 30-liter jar fermenters each containing 15 liters of the same sterilized medium.

The inoculated media were cultured at 27°C with stirring at 250 rpm under aeration with 15 liters of sterile air per minute. After 3 days of culture, the cultured broths were combined, filtered and washed with water. Thus was obtained 61 liters of the filtrate including the washings. 1 kg of activated carbon was added to the filtrate, and the mixture was stirred to adsorb foroxymithine to the activated carbon. Upon filtration, the activated carbon was washed thoroughly with water, and placed in 15 liters of a 50% aqueous solution of acetone. The solution was stirred to elute foroxymithine from the activated carbon. Then, the solution was filtered, and the filtrate was concentrated under reduced pressure to obtain 7.6 liters of the concentrate. 7.6 liters of this concentrate was absorbed to a 1.15 liter column of DEAE-Sephadex ®A25 ($OH^-$ type), washed with deionized water, and eluted with 4 liters of a 0.1N aqueous solution of sodium chloride. The eluate was neutralized with 6N HCl to a pH of 7.0, and concentrated to dryness under reduced pressure to obtain 61.5 g of a crude powder. The crude powder was dissolved in 300 ml of deionized water, and the solution was adjusted to pH 3.0 with 6N hydrochloric acid. The solution was adsorbed to a 1.5 liter column of reversed-phase silica gel (Kieselgel 60 , silanized, a product of Merck) equilibrated with a 0.4% aqueous solution of acetic acid, and was then developed with a 0.4% aqueous solution of acetic acid. Active fractions were combined to obtain 470 ml of crude fraction. 100 ml taken from this 470 ml active fraction was concentrated to 20 ml under reduced pressure. The concentrate was absorbed by means of PrepLC/System 500 (a product of Waters), to a reversed-phase HPLC column (PrepPAK-500/$C_{15}$) which had been equilibrated with a 1% aqueous solution of acetic acid. The column was developed with a 1% aqueous solution of acetic acid at a flow rate of 50 ml/minute with the use of the same HPLC system. Active fractions were combined and concentrated to dryness under reduced pressure to obtain 6.35 g of a crude powder. The crude powder was dissolved in a 0.4% aqueous solution of acetic acid, and the solution divided into 10 equal parts was adsorbed to a preparative HPLC column, Nucleosil® 5C18 (described previously), with one of said parts being adsorbed at a time. The column had been equilibrated with a 10:90 mixture of methanol and a 0.4% aqueous solution of acetic acid, the adsorbate was eluted with the same mixture at a flow rate of 6 ml per minute. Fractions were collected in amounts of 6 ml each, and active fractions were combined. The

combined fraction was concentrated, and lyophilized after addition of water, thereby obtaining 3.66 g of a white powder ($IC_{50}$ = 7 µg/ml). The thus obtained substance had exactly the same physicochemical properties as those of foroxymithine dihydrate which is produced by Streptomyces nitrosporeus MG329-CF56 (FERM P-7244). With Streptomyces zaomyceticus MG325-CF7 (FERM P-7243), the yield of foroxymithine was 3.66 g of foroxymithine dihydrate from 12.8 liters of the culture broth filtrate, as noted above. Thus, it has become clear that foroxymithine-producing strains of the genus Streptomyces include zaomyceticus in addition to nitrosporeus.

CLAIMS:

1. The compound having the formula

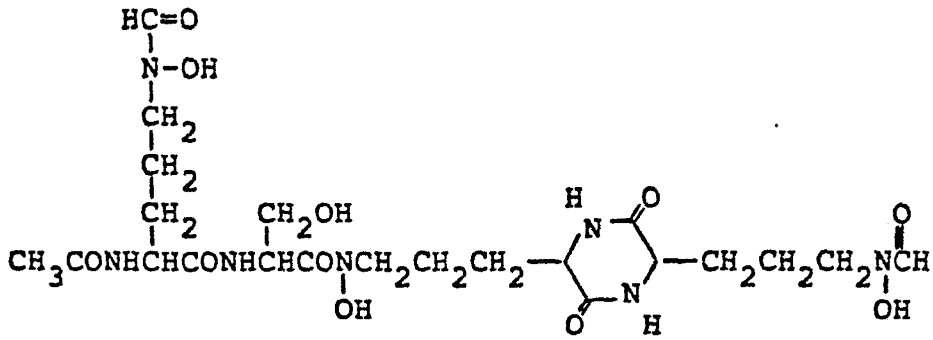

of pharmaceutically acceptable salts or esters thereof.

2. A process for producing a compound of the formula

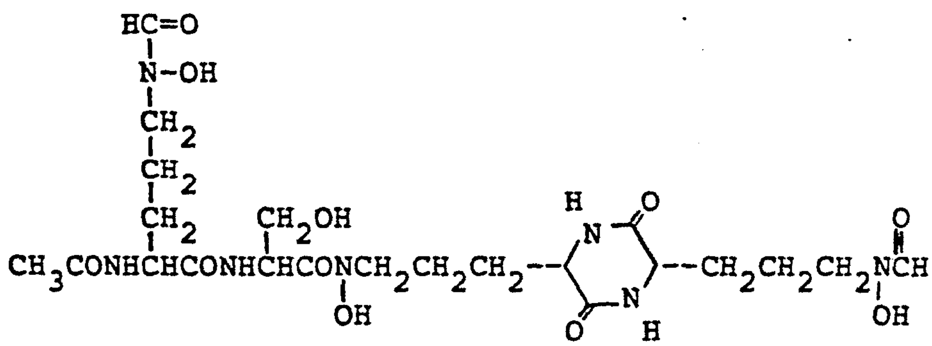

which comprises cultivating a foroxymithine producing microorganism of the genus Streptomyces in a usual manner, in a nutrient medium to produce and accumulate foroxymithine in the culture medium, and recovering it from the culture by filtration and adsorption in a customary manner.

3. The process as claimed in claim 2 wherein the foroxymithine producing microorganism is Streptomyces nitrosporeus FERM P-7244 or Streptomyces zaomyceticus FERM P-7243.

4. The process as claimed in claims 2 and 3 wherein the cultivation is carried out in a nutrient medium containing sources of carbon and nitrogen and inorganic nutrient salts at temperatures of from 25 to 35°C and the compound of claim 1 is isolated from the culture filtrate by adsorption at a customary adsorbent, followed by elution with a suitable solvent, and purification by chromatography.

5. The process as claimed in claim 4 wherein the nutrient medium comprises glycol, cotton seed meal, sodium chloride and L-asparagine monohydrate, the adsorbent is activated carbon, the eluent is an aqueous solution of acetone, and the obtained compound is purified by column chromatography, followed by reversed-phase high-performance liquid chromatography.

6. A pharmaceutical composition comprising as active ingredient the compound claimed in claim 1 or a physiologically acceptable salt or ester thereof in association with a pharmaceutically acceptable carrier.

7. Use of the compound as claimed in claim 1 or a physiologically acceptable salt or ester thereof for the manufacture of a medicament having antihypertensive activity.

8. The microorganism Streptomyces nitrosporeus FERM P 7244.

9. The microorganism Streptomyces zaomyceticus FERM P 7243.

<u>CLAIMS</u> for the contracting state AT:

1. A process for producing a compound of the formula

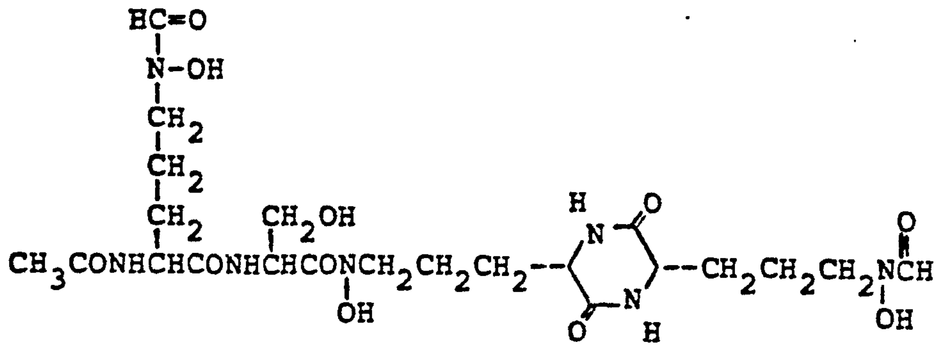

which comprises cultivating a foroxymithine producing microorganism of the genus <u>Streptomyces</u> in a usual manner, in a nutrient medium to produce and accumulate foroxymithine in the culture medium, and recovering it from the culture by filtration and adsorption in a customary manner.

2. The process as claimed in claim 1 wherein the foroxymithine producing microorganism is Streptomyces nitrosporeus FERM P-7244 or Streptomyces zaomyceticus FERM P-7243.

3. The process as claimed in claims 1 and 2 wherein the cultivation is carried out in a nutrient medium containing sources of carbon and nitrogen and inorganic nutrient salts at temperatures of from 25 to 35°C and the compound of claim 1 is isolated from the culture filtrate by adsorption at a customary adsorbent, followed by elution with a suitable solvent, and purification by chromatography.

4. The process as claimed in claim 3 wherein the nutrient medium comprises glycol, cotton seed meal, sodium chloride and L-asparagine monohydrate, the adsorbent is activated carbon, the eluent is an aqueous solution of acetone, and the obtained compound is purified by column chromatography, followed by reversed-phase high-performance liquid chromatography.

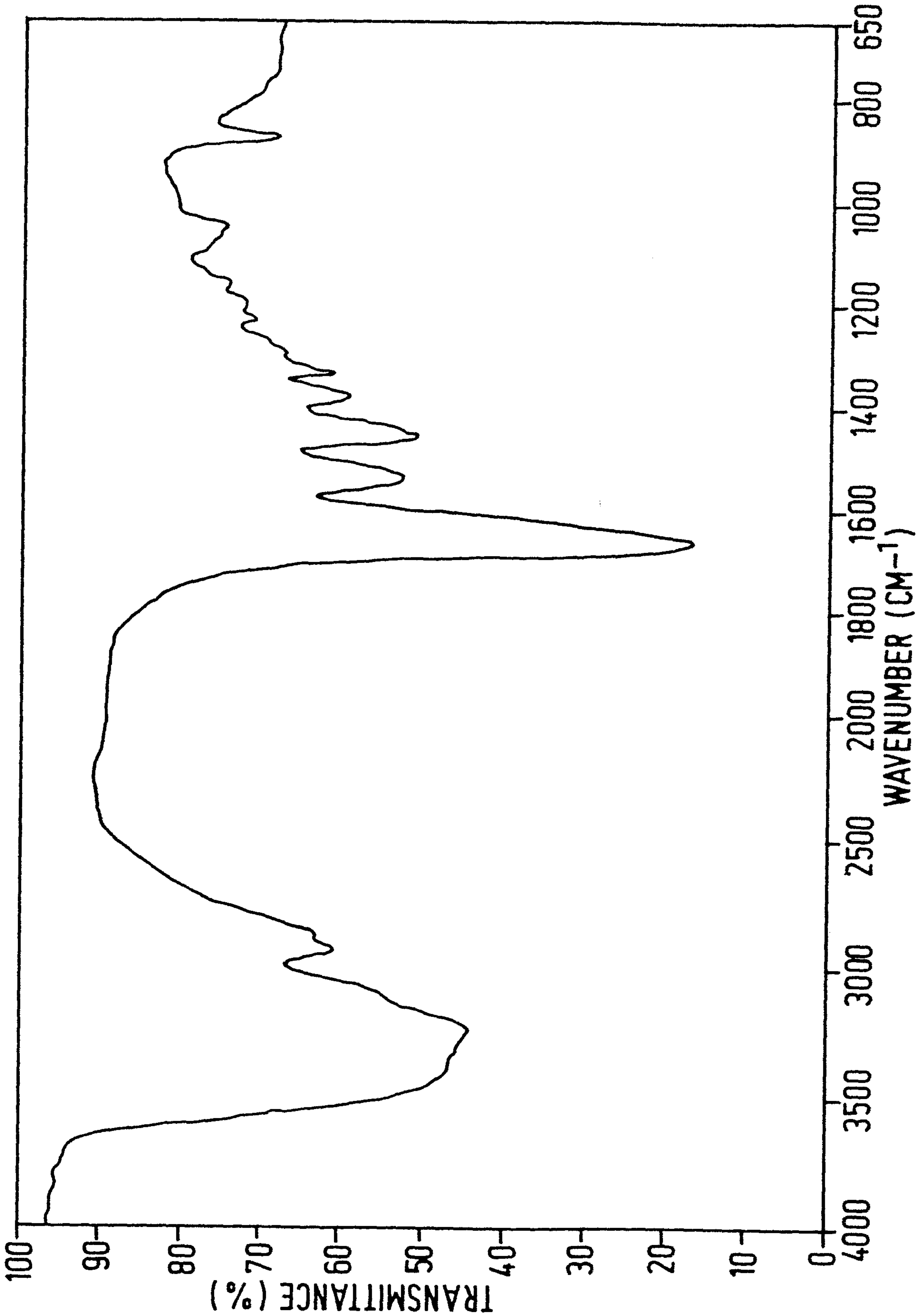

TRANSMITTANCE (%)
100
90
80
70
60
50
40
30
20
10
0
WAVENUMBER (CM$^{-1}$)
4000
3500
3000
2500
2000
1800
1600
1400
1200
1000
800
650